# EUROPEAN PATENT APPLICATION

(11) **EP 3 851 080 A1**
(43) Date of publication of application: **21.07.2021**
(21) Application number: 19858881.6
(22) Date of filing: 31.05.2019
(51) Int. Cl.: A61F 2/95, A61F 2/962

(54) **BODY OF A DEVICE FOR POSITIONING A CORONARY STENT OR CORONARY BALLOON IN CORONARY ARTERIES**

(30) Priority: 13.09.2018 RU 2018132608
(71) Applicant: Seven Sons Ltd. R.N. 515985570 (The "Company"), 67443 Tel Aviv (IL)
(72) Inventor: TRAPEZNIKOV, Vladimir Borisovich, Moscow, 121165 (RU)
(74) Representative: Loven, Keith James
(86) International application number: PCT/IB2019/000587
(87) International publication number: WO 2020/053643

(57) **Abstract**

A body of a device for positioning a coronary stent or a coronary balloon within coronary arteries is intended for use in medicine. The body is formed of a front part and a rear part each representing two identical halves. The rear body part is connected to the front part in an overlapping manner. The front body part represents, on the one side, a truncated part having a tubular extension with a cutout for connection to a Y-connector and, on the other side, successively passes into a cylindrical part, a cylindrical smooth part of a smaller diameter and a runner guide. In its cylindrical front part on both sides, the body is provided with rectangular holes for retainer lugs with point protrusions in each for holding the retainer lugs in an extreme position and the retainer itself in a locked position. A cylindrical slot with point elements is provided circumferentially around the cylindrical smooth front body part on the runner guide side. The inside surface of the rear body part is formed as a smooth surface and as a circumferentially double-threaded surface. The smooth surface of the rear body part is housed over the cylindrical smooth front body part, and the inside surface provided with the circumferential double-thread for engaging with the runner protrusions is housed over the runner. Between the smooth inside surface and the surface provided with the inner double-thread in the rear body part, there is circumferentially provided a cylindrical protrusion with point elements for engaging with the cylindrical slot in the cylindrical smooth part of the front body part on the runner guide side to allow the cylindrical rear body part to be rotatable around its axis. The outside rear body part is provided with horizontally extending smooth transparent stripes alternating with pebbled surface stripes. Through-holes for introducing the coronary stent or coronary balloon delivery system inside the device housed in the body are formed in the tubular extension of the front body part provided with cutout for the coronary guide and in the end surface of the rear body part. A chamfer is formed in the end surface of the rear body part.

## Description

This invention relates to a body of a device intended for use in medicine for positioning, in a maximum accurate, fast and safe manner, a coronary stent or a coronary balloon on a delivery system in case of endovascular coronary stenting.

In the medical equipment market, there are known devices for positioning a stent in the coronary arteries of the same applicant (RU 2598798, RU 2652732, RU 2650038, RU 2661092), the body of which differs from the presently claimed device having the following advantages:
- optimized positioning a coronary stent or a coronary balloon by increasing the body length and thereby the range of displacement of the locked delivery system,
- improved operating efficiency as a result of increasing the area of engagement with fingers of a rear body part and positioning one's hands as close as possible to each other owing to a structure wherein the rear body part is configured to overlap a front part,
- improved safety and stability of the device operation by providing point protrusions within holes for retainer lugs in the front body part, said protrusions allowing the retainer to be kept in a locked position to prevent thereby possible spontaneous release of the coronary stent or coronary balloon delivery system when locked,
- improved operating safety and stability owing to stripes with a pebbled surface horizontally extending along the rear body part, which improves engagement between the body surface and the surgeon's fingers,
- possibility of visual inspection of the mechanism operation inside the body owing to transparent stripes alternating with pebbled stripes horizontally extending along the rear body part,
- reduced friction and improved rotation quality owing to mutually rotatable point elements provided on the body surfaces,
- enabling a secure and safe locking of the device when attached to a Y-connector owing to providing the front part of the device with a tubular extension,
- enabling a smooth and convenient contact between the rear body part end and the surgeon's hand owing to a chamfer,
- simplified structure and lower production costs owing to providing identical halves of the rear body part by using a double-thread on a cylindrical inside surface of the rear body part.

The claimed invention is industrially applicable.

The subject of this invention is a body of a device for positioning a coronary stent or a coronary balloon within coronary arteries.

The body includes a retainer with thickened lugs, a runner having a hole for receiving a delivery system, with protrusions for engaging with an inner double-thread on a cylindrical rear body part and with protrusions for moving along guides inside the body, on the one side, and with a cap having a hole for receiving the delivery system with a rubber bush having a hole for receiving the delivery system with a cone-shaped and end surface inside matching the same surfaces inside the cap and at the runner end, housed in the cap with a thread for threading on the runner, on the other side.

A mechanism housed in the body is a subject matter of an independent patent for invention RU 2661092) owned by the present applicant and is cited here in only for illustration purpose and understanding of the claimed utility model.

The body is formed of a front part and a rear part each representing two identical halves connected to each other. The rear body part is connected to the front part in an overlapping manner.

The front body part represents, on the one side thereof, a truncated part having a tubular extension and, on the other side, successively passes into a cylindrical part, a cylindrical smooth part of a smaller diameter and a runner guide.

The truncated front body part is adapted for being held with the surgeon's left hand fingers and is provided with recesses allowing the surgeon to lock the device in position, said also serving as a site for locking a coronary guide. The recesses are provided with a pebbled surface.

The truncated front body part is intended for being connected to a Y-connector and is provided with a tubular extension with a cutout for the coronary guide. The tubular body extension is inserted into a hole in the Y-connector and the coronary guide extends through the cutout in the tube to come out at on the outside of the truncated front body part where the recess begins.

Such structure allows the device to be accurately and firmly connected to the Y-connector and any spontaneous displacement of the device body relative to Y-connector to be prevented while positioning the coronary stent or the coronary balloon.

Such structure also reduces the load on the surgeon fingers, and facilitates and simplifies the surgery.

As an embodiment, the truncated front body part can be made without tubular extension and can be not intendent for being connected to the Y-connector.

In its cylindrical front part on both sides, the body is provided with rectangular holes for retainer lugs. Each of the rectangular holes, on the one side, at a distance equal to the width of the retainer lug is provided with point protrusions for holding the retainer lugs in an extreme position and the retainer itself in a locked position.

Circumferentially around the cylindrical smooth front body part on the runner guide side, there is provided a cylindrical slot with point elements for connecting to the respective protrusion with point elements, provided circumferentially around the cylindrical smooth inside surface of the rear body part on the double-threaded surface side.

The cylindrical rear body part is formed as a smooth surface on the inside and as a circumferentially double-threaded surface.

The cylindrical smooth inside surface of the rear body part on the retainer side is provided with circumferentially housed point elements for reducing the friction of mutually rotatable body parts.

Between the smooth inside surface and the surface provided with the inner double-thread in the rear body part, there is circumferentially provided a cylindrical protrusion with point elements for engaging with the cylindrical slot in the cylindrical smooth part of the front body part on the runner guide side to allow the cylindrical rear body part to be rotatable around its axis.

The outside of the rear body part is provided with horizontally extending smooth transparent stripes alternating with pebbled surface stripes.

As an embodiment, the outside of the rear body part may be completely provided with a pebbled surface.

When assembling the device, the smooth surface of the rear body part is housed over the cylindrical smooth front body part and the inside provided with the circumferential double-thread for engaging with the runner protrusions is housed over the runner.

The rear body part is housed on top of the front body part in an overlapping manner over the cylindrical smooth part with retainer inside and over the runner.

A chamfer is formed in the end surface of rear body part to provide a smooth and convenient contact of the end surface with the surgeon's palm.

Through-holes for introducing the coronary stent or coronary balloon delivery system inside the device housed in the body are formed in the tubular extension of the front body part provided with cutout for the coronary guide and in the end surface of the rear body part.

The body is made of plastic by injection molding and is formed of the front and rear parts each representing two identical halves.

Each half of the truncated front part, cylindrical part and smaller-diameter cylindrical smooth part of the front body part and each halve of the rear body part are molded integrally.

As an embodiment, the halves of the rear body part may be provided with an offset single-thread on the inside to form a common threaded surface When assembling the device.

The halves are produced separately and connected to each other once the mechanism is assembled using tapered protrusions and matching tapered holes formed on the edges of the front and rear body inner parts.

When assembling the device, the rubber bush with the hole for receiving the delivery system and the cap with the hole for receiving the delivery system are first successively housed in the retainer on the thickened side, the runner with the hole for receiving the delivery system is housed on the opposite side of the retainer and the cap with the rubber bush inside is threaded into said hole. Then, a tube is introduced into the through-hole formed in the bush, cap and runner for arranging therein the coronary stent or coronary balloon delivery system.

The retainer with the rubber bush, cap and runner is housed inside the front body part with the thickened part of the retainer accommodated in the cylindrical body part with side holes for the retainer lugs and the rest of the retainer is accommodated in the cylindrical smooth part of the front body part.

The halves of the front body part are connected using tapered protrusions and matching tapered holes formed in the front body part whereafter the cylindrical smooth part of the front body part is housed in the halves of the rear body part and the cylindrical protrusion with point elements is housed in the cylindrical slot formed in the cylindrical smooth part of the front body part, wherein the halves of the rear body part are connected similarly to those of the front body part using tapered protrusions and matching tapered holes in the rear body part to form thereby an overlapping connection with the front body part.

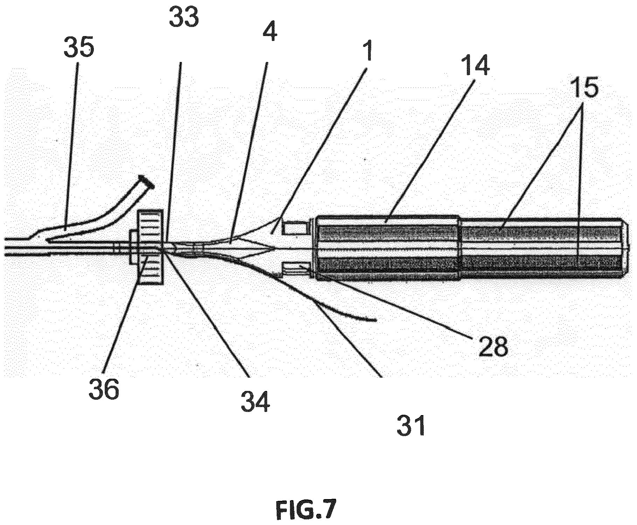

Provided below is a list of the attached drawings.

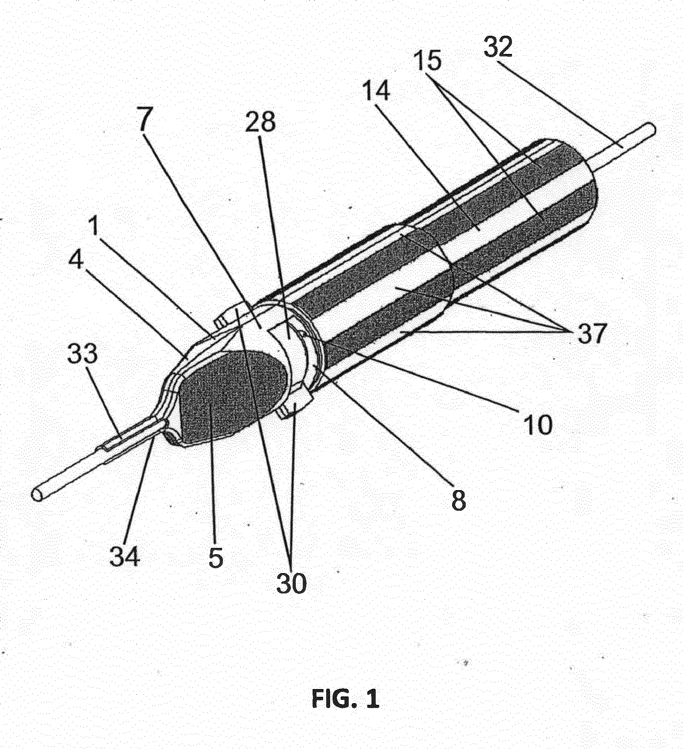

Fig. 1 is a general view of the body, showing:
   1 - front body part,
   4 - truncated part of the front body part,
   5 - finger recesses with the pebbled surface,
   7 - cylindrical part of the front body part,
   8 - holes in the cylindrical front body part for the retainer lugs,
   10 - point protrusion for holding the retainer lugs,
   14 - rear body part,
   15 - pebbled surface,
   28 - retainer,
   30 - retainer lugs,
   32 - delivery system tube,
   33 - tubular body extension,
   34 - cutout for the coronary guide,
   37 - smooth transparent stripe on the outside of the rear body part.

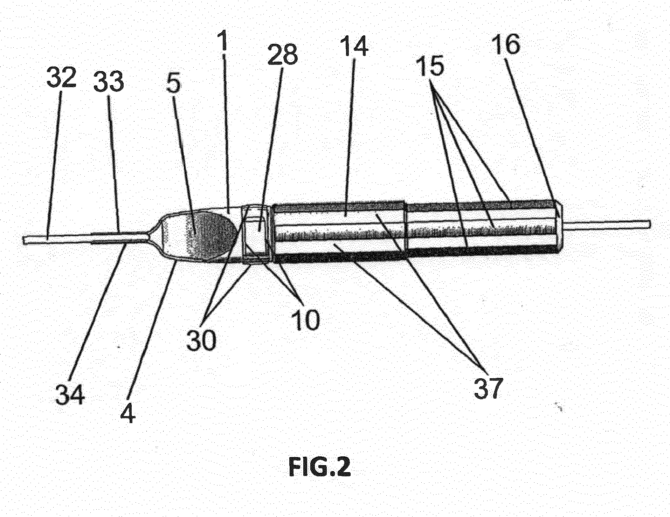

Fig. 2 is a side view of the body, showing:
   1 - front body part,
   4 - truncated part of the front body part,
   5 - finger recesses with pebbled surface,
   10 - point protrusion for holding the retainer lugs,
   14 - rear body part,
   15 - pebbled surface,
   16 - chamfer in the rear end body part,
   28 - retainer,
   30 - retainer lugs,
   32 - tube for the delivery system,
   33 - tubular body extension,
   34 - cutout in the tube for the coronary guide,
   37 - smooth transparent stripe on the outside of the rear body part.

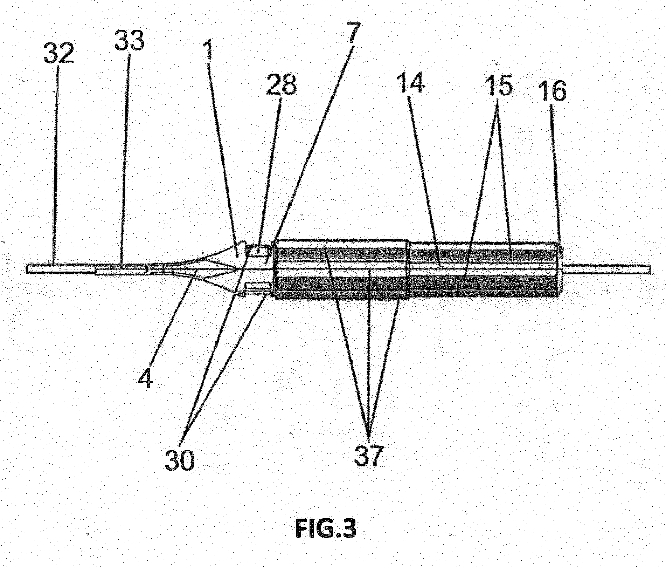

Fig. 3 is a top view of the body, showing:
   1 - front body part,
   4 - truncated part of the front body part,
   7 - cylindrical part of the front body part,
   14 - rear body part,
   15 - pebbled surface,
   16 - chamfer of the rear end body part,
   28 - retainer,
   30 - retainer lugs,
   32 - delivery system tube
   33 - tubular body extension,
   37 - smooth transparent stripe on the outside of the rear body part.

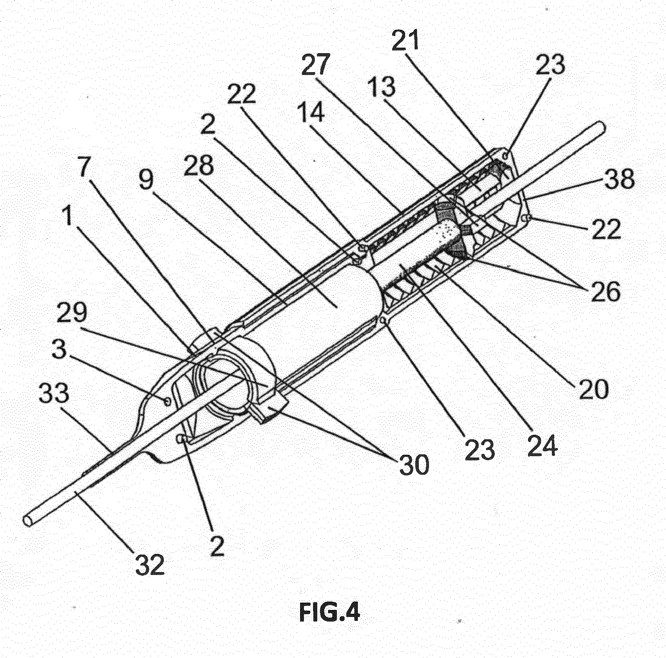

Fig. 4 is cross-sectional view of the body with the device, showing:
   1 - front body part,
   2 - tapered protrusion on the front body part,
   3 - tapered hole in the front body part,
   7 - cylindrical part of the front body part,
   9 - cylindrical smooth part of the front body part
   13 - runner guides,
   14 - rear body part,
   20 - internal double-thread internal in the rear body part for engaging with the runner,
   21 - through-hole for the coronary stent or coronary balloon delivery system,
   22 - tapered protrusion on the rear body part,
   23 - tapered hole in the rear body part,
   24 - runner,
   26 - runner protrusion for engaging with the thread in the inside of the of the rear body part,
   27 - protrusion for the front body part guides,
   28 - retainer,
   29 - retainer thickening,
   30 - retainer lugs,
   32 - delivery system tube,
   33 - tubular body extension.

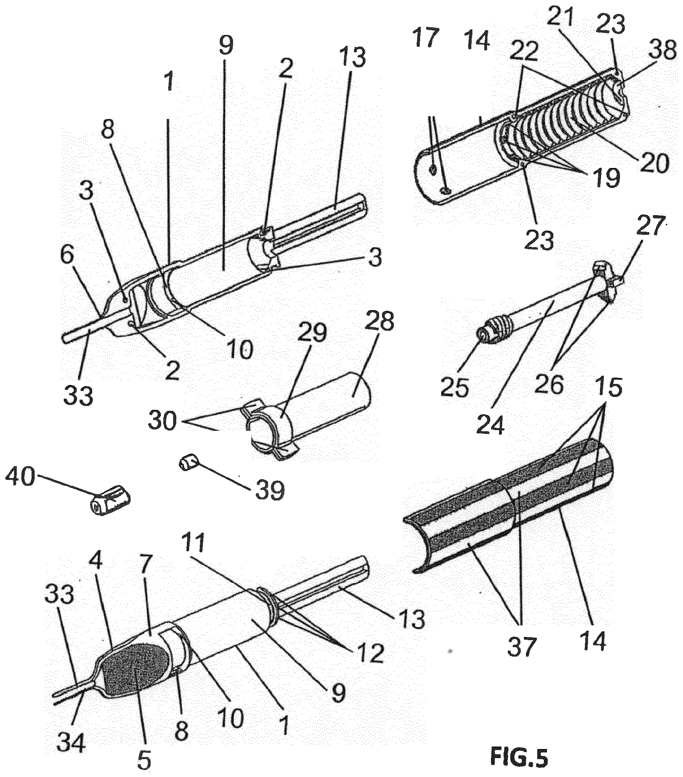

Fig. 5 is a detached view of the body with the device, showing:
   1 - front body part,
   2 - tapered protrusion on the front body part,
   3 - tapered hole in the front body part,
   4 - truncated part of the front body part,
   5 - finger recesses with pebbled surface,
   6 - delivery system receiving hole in the front body part,
   7 - cylindrical part of the front body part,
   8 - holes in the cylindrical front body part for retainer lugs,
   9 - cylindrical smooth part of the front body part,
   10 - point protrusion for holding the retainer lugs,
   11 - cylindrical slot in the cylindrical smooth part of the front body part,
   12 - point elements of the cylindrical slot in the front body part,
   14 - runner guides,
   14 - rear body part,
   15 - pebbled surface,
   17 - point elements of the smooth inner rear body part,
   18 - cylindrical protrusion,
   19 - cylindrical protrusion point elements,
   20 - internal double-thread in the rear body part for engaging with the runner,
   21 - through-hole for the coronary stent or coronary balloon delivery system,
   22 - tapered protrusion on the rear body part,
   24 - tapered hole in the rear body part,
   24 - runner,
   25 - runner hole for the coronary stent or coronary balloon delivery system,
   26 - runner protrusion for engaging with the thread in the inside of the rear body part,
   27 - protrusion for the front body part guides,
   28 - retainer,
   29 - retainer thickening,
   30 - retainer lugs,
   33 - tubular body extension,
   34 - cutout in the tube for the coronary guide,
   37 - smooth transparent stripe on the outside of the rear body part,
   38 - end surface of the rear body part,
   39 - rubber bush,
   40 - cap.

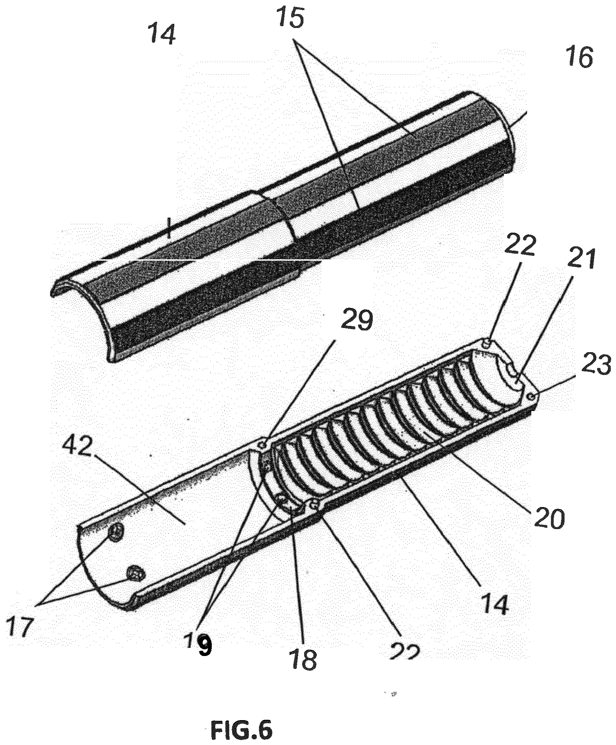

Fig. 6 is a general view of the rear body part halves, showing:
   14 - rear body part,
   15 - pebbled surface,
   16 - chamfer in the rear end body part,
   17 - point elements of the smooth inner rear body part,
   18 - cylindrical protrusion,
   19 - cylindrical protrusion point elements,
   20 - internal double-thread in the rear body part for engaging with the runner,
   21 - through-hole for the coronary stent or coronary balloon delivery system,
   22 - tapered protrusion on the rear body part,
   24 - tapered hole in the rear body part,
   42 - smooth internal surface of the rear body part
Fig. 7 is a general view of the device with a Y-connector, showing:
   1 - front body part,
   4 - truncated part of the front body part,
   14 - rear body part,
   15 - pebbled surface,
   28 - retainer,
   31 - coronary guide,
   33 - tubular body extension,
   34 - cutout in the tube for the coronary guide,
   35 - Y-connector,
   36 -Y-connector hole for introducing the device tube.
Fig. 8 - is a general view of the rear body part with offset thread, showing:
   14 - rear body part,
   41 - rear body part with offset threaded inside surface.
Fig. 9 is a general view of the front body part once connected to the rear part, showing:
   1 - front body part,
   5 - finger recesses with pebbled surface,
   14 - rear body part,
   28 - retainer,
   33 - tubular body extension,
   34 - cutout in the tube for the coronary guide.
Fig. 10 is a general view of the body with a continuously pebbled surface of its rear part, showing:

1 - front body part,
5 - finger recesses with pebbled surface,
14 - rear body part,
28 - retainer,
38 - end surface rear body part,
43 - continuously pebbled surface of the rear body part.
Fig. 11 is a general view of the body having its front part without tubular extension, showing:
   1 - front body part,
   4 - truncated part of the front body part,
   5 - finger recesses with pebbled surface,
   6 - delivery system receiving hole in the front body part,
   7 - cylindrical part of the front body part,
   14 - rear body part,
   28 - retainer.
Fig. 12 is a general view front body part without tubular extension once connected to the rear part, showing:
   1 - front body part,
   4 - truncated part of the front body part
   5 - finger recesses with pebbled surface,
   6 - delivery system receiving hole in the front body part,
   7 - cylindrical part of the front body part,
   14 - rear body part,
   28 - retainer.

Provided below is the description of the invention with reference to the attached drawings.

The body is formed of a front body part 1 (Fig. 1, Fig. 2, Fig. 3, Fig. 4, Fig. 5, Fig. 7) and a rear body part 14 (Fig. 1, Fig. 2, Fig. 3, Fig. 4, Fig. 5, Fig. 6, Fig. 7) each representing two identical halves. The rear body part 14 (Fig. 1, Fig. 2, Fig. 3, Fig. 4, Fig. 5, Fig. 6, Fig. 7, Fig. 8) is connected to the front body part 1 (Fig. 1, Fig. 2, Fig. 3, Fig. 4, Fig. 5) in an overlapping manner.

The front body part 1 (Fig. 1, Fig. 2, Fig. 3, Fig. 4, Fig. 5, Fig. 7) represents a truncated part 4 (Fig. 1, Fig. 2, Fig. 3, Fig. 5, Fig. 7), on the one side, with a tubular extension 33 (Fig. 5, Fig. 7) successively passing into a cylindrical part 7 (Fig. 1, Fig. 2, Fig. 3, Fig. 5), a cylindrical smooth part of a smaller diameter 9 (Fig. 5) and a runner guide 13 (Fig. 4, Fig. 5), on the other side.

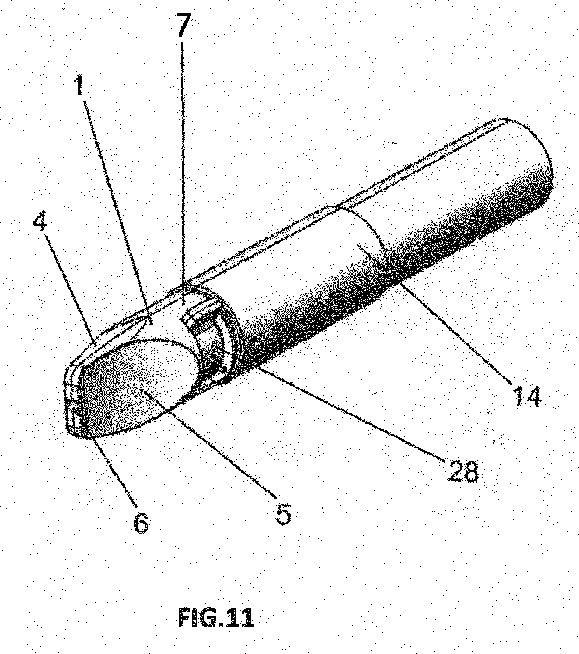

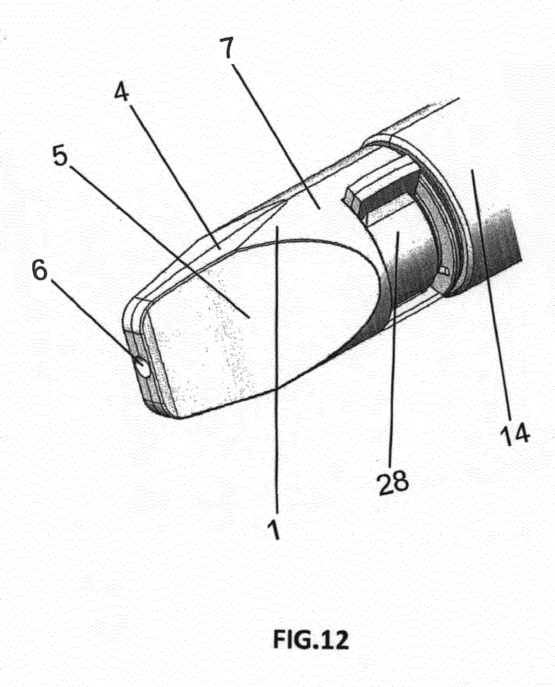

As an embodiment, the truncated front body part 4 (Fig. 11, Fig. 12) may have no without tubular extension.

The truncated front part 4 (Fig. 1, Fig. 2, Fig. 3, Fig. 5, Fig. 7) of the body 1 (Fig. 1, Fig. 2, Fig. 3, Fig. 4, Fig. 5, Fig. 7) is adapted for being held with the surgeon's left hand fingers and is provided with recesses 5 (Fig. 1, Fig. 2, Fig. 5) allowing the surgeon to lock the device in position, said recessed 5 (Fig. 1, Fig. 2, Fig. 5) also serving as a locking site for the coronary guide. The recesses 5 (Fig. 1, Fig. 2, Fig. 5) for the surgeon fingers in truncated front body part 4 (Fig. 1, Fig. 2, Fig. 3, Fig. 5, Fig. 7) are provided with a pebbled surface.

The truncated front body part 4 (Fig. 1, Fig. 2, Fig. 3, Fig. 5, Fig. 7) is intended for connection to a Y-connector 35 (Fig. 7) and is provided with the tubular extension 33 (Fig. 5, Fig. 7) with a cutout 34 (Fig. 5, Fig. 7) for the coronary guide 31 (Fig. 7). The tubular body extension 33 (Fig. 5, Fig. 7) is inserted into the Y-connector 35 (Fig. 7) and the coronary guide 31 (Fig. 7) extends through the cutout 34 (Fig. 5, Fig. 7) in the tube 33 (Fig. 5, Fig. 7) to come out on the outside of the truncated front body part 4 where the recess 5 (Fig. 1, Fig. 2, Fig. 7).

As an embodiment of the truncated front body part 4 without tubular extension (Fig. 11, Fig. 12), a tube 32 (Fig. 4) for accommodating the coronary stent or coronary balloon delivery system inside the device is provided in an opening 6 in the truncated front part 4 of the body (Fig. 11, Fig. 12) and in a through-hole 21 in the end surface 38 (Fig. 4, Fig. 5) of the rear body part 14 (Fig. 1, Fig. 2, Fig. 3, Fig. 4, Fig. 5, Fig. 6, Fig. 7).

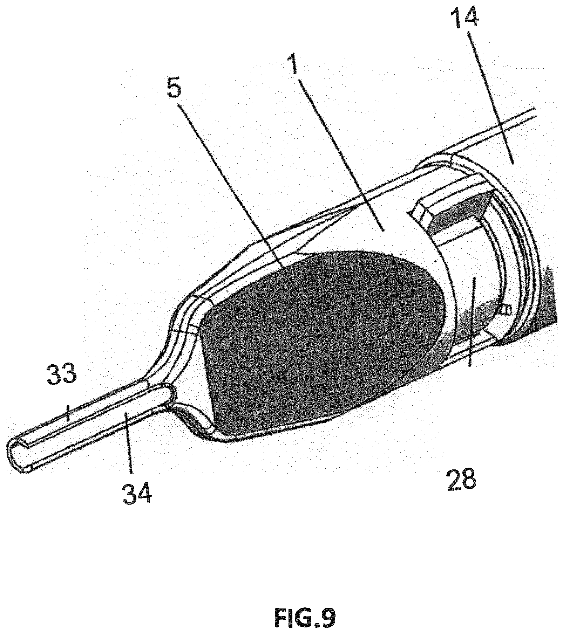

In its cylindrical front part 7 (Fig. 1, Fig. 3, Fig. 5), the body 1 (Fig. 1, Fig. 2, Fig. 3, Fig. 4) is provided on both sides with rectangular holes 8 (Fig. 1, Fig. 5) for years 30 (Fig. 1, Fig. 4, Fig. 5) of a retainer 28 (Fig. 4, Fig. 5, Fig. 7). Each of the rectangular holes 8 (Fig. 1, Fig. 2, Fig. 3, Fig. 4, Fig. 5, Fig. 9), on the one side, at a distance equal to the width of the lugs 30 (Fig. 1, Fig. 4, Fig. 5) of the retainer 28 (Fig. 4, Fig. 5, Fig. 7), accommodates two point protrusions 10 (Fig. 1, Fig. 2, Fig. 5) for holding the lugs 30 (Fig. 1, Fig. 4, Fig. 5) of the retainer 28 (Fig. 4, Fig. 5, Fig. 7) in an extreme position and the retainer itself 28 (Fig. 4, Fig. 5, Fig. 7) in a locked position.

Circumferentially around the cylindrical smooth front body part 9 (Fig. 5) of the front body part, on the side opposite to the truncated front body part 4 (Fig. 1, Fig. 2, Fig. 3, Fig. 5, Fig. 7), there is a formed a cylindrical slot 11 (Fig. 5) with point elements 12 (Fig. 5) for connecting to the respective protrusion 18 (Fig. 5) with point elements 19 (Fig. 5, Fig. 6), provided circumferentially around the cylindrical smooth inside surface 42 (Fig. 6) of the rear body part 14 (Fig. 1, Fig. 2, Fig. 3, Fig. 4, Fig. 5, Fig. 6, Fig. 7) on the double-threaded surface side 20 (Fig. 6).

The cylindrical rear body part 14 (Fig. 1, Fig. 2, Fig. 3, Fig. 4, Fig. 5, Fig. 6, Fig. 7) on the inside is formed circumferentially as a smooth surface 42 (Fig. 6) and the double-threaded surface 20 (Fig. 6).

Point elements 17 (Fig. 5, Fig. 6) are formed circumferentially on the smooth inside surface 42 (Fig. 6) of the rear body part 14 (Fig. 1, Fig. 2, Fig. 3, Fig. 4, Fig. 5, Fig. 6, Fig. 7) on the retainer 28 (Fig. 4, Fig. 5, Fig. 6) side.

Between the smooth inside surface 42 (Fig. 6) and inner double-thread surface 20 (Fig. 5, Fig. 6) of the rear body part 14 (Fig. 1, Fig. 2, Fig. 3, Fig. 4, Fig. 5, Fig. 6, Fig. 7), there is formed a cylindrical protrusion 18 (Fig. 5, Fig. 6) with point elements 19 (Fig. 5, Fig. 6) for engaging with the cylindrical slot 11 (Fig. 5) in the cylindrical smooth part 9 of the front body (Fig. 4, Fig. 5) on the runner guide 13 side (Fig. 4, Fig. 5) to allow the cylindrical rear body part 14 (Fig. 1, Fig. 2, Fig. 3, Fig. 4, Fig. 5, Fig. 6, Fig. 7) to be rotatable around its axis.

The outside of the rear body part 14 (Fig. 1, Fig. 2, Fig. 3, Fig. 4, Fig. 5, Fig. 6, Fig. 7) is provided with horizontally extending smooth transparent stripes 37 Fig. 1, Fig. 2, Fig. 3) alternating with pebbled surface stripes 15 (Fig. 1, Fig. 2, Fig. 3).

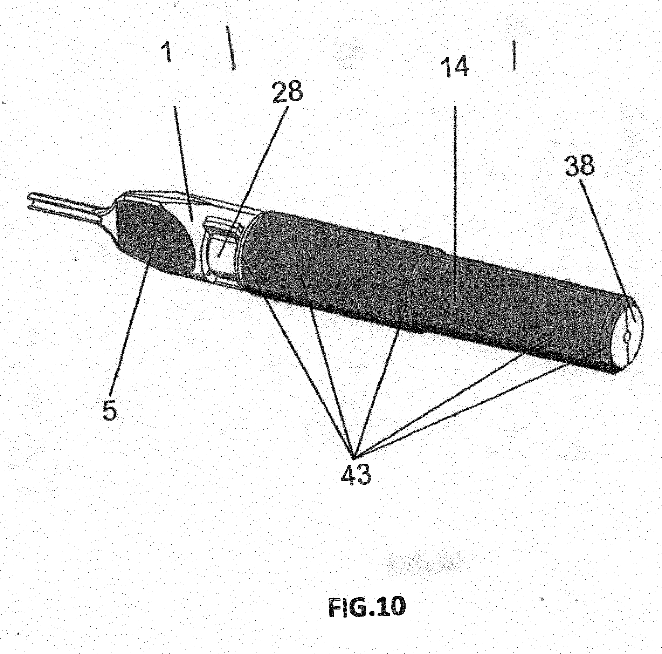

As an embodiment, the outside of the rear body part 14 (Fig. 10) may have a continuously pebbled surface 43 (Fig. 10).

The rear body part 14 (Fig. 1, Fig. 2, Fig. 3, Fig. 4, Fig. 5, Fig. 6, Fig. 7) is housed in an overlapping manner on top of the front body part 1 (Fig. 4) over the cylindrical smooth part 9 (Fig. 5) with the retainer 28 (Fig. 4, Fig. 5, Fig. 7) inside and over the runner 24 (Fig. 4, Fig. 5).

A chamfer 16 (Fig. 2, Fig. 3, Fig. 6) enabling a smooth and convenient contact of the body end part with the surgeon's palm is formed in the end surface 38 (Fig. 4, Fig. 10) of the rear body part.

Through-holes 21 (Fig. 4, Fig. 5, Fig. 6) for introducing the coronary stent or coronary balloon delivery system inside the device housed inside the body are provided in the tubular extension 33 (Fig. 5, Fig. 7, Fig. 9) of the front body part provided with the cutout 34 (Fig. 5, Fig. 7, Fig. 9) for the coronary guide 31 (Fig. 7) and in the end surface 38 (Fig. 4, Fig. 5) of the rear body part 14 (Fig. 1, Fig. 2, Fig. 3, Fig. 4, Fig. 5, Fig. 6, Fig. 7).

The body is made of plastic by injection molding and is formed of a front part 1 and a rear part 14 (Fig. 1, Fig. 2, Fig. 3, Fig. 5, Fig. 7) each representing two identical halves.

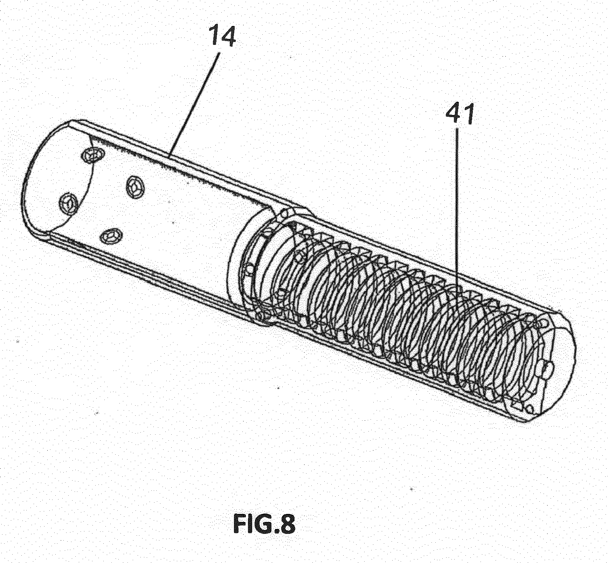

As an embodiment, the halves may be provided with an offset single-thread 41 (Fig. 8) to form a common threaded surface when assembling the device.

The halves are made separately and connected to each other, once the mechanism is assembled, using tapered protrusions 2, 22 (Fig. 4, Fig. 5) and matching tapered holes 3, 23 (Fig. 4, Fig. 5) formed along the edges of the inner body part.

When assembling the device, the rubber bush 39 (Fig. 5) with the hole for receiving the delivery system and the cap 40 (Fig. 5) with the hole for receiving the delivery system are first successively housed in the retainer 28 (Fig. 4, Fig. 5) on the thickened side and the runner 24 (Fig. 4, Fig. 5) with the hole for receiving the delivery system is housed on the opposite side of the retainer 28 (Fig. 4, Fig. 5) and the cap 40 (Fig. 5) with the rubber bush 39 (Fig. 5) inside is threaded into said hole. Then, the tube 32 (Fig. 1, Fig. 4) is introduced into the through-hole formed in the bush 39, cap 40 and runner 24 for arranging therein the coronary stent or coronary balloon delivery system.

The retainer 28 (Fig. 4, Fig. 5) with the rubber bush 39 (Fig. 5), cap 40 (Fig. 5) and runner (Fig. 4, Fig. 5) is housed inside the front body part 1 (Fig. 4) with the thickened part of the retainer 29 (Fig. 4, Fig. 5) accommodated in the cylindrical body part 7 (Fig. 4) with the side holes 8 (Fig. 1, Fig. 4) for the lugs 30 (Fig. 1, Fig. 4) of the retainer 28 (Fig. 1, Fig. 4) and the rest of the retainer is accommodated in the cylindrical smooth part of the front body part 9 (Fig. 4).

The halves of the front body part are connected using tapered protrusions 2 (Fig. 4, Fig. 5) and matching tapered holes 3 (Fig. 4, Fig. 5) formed in the front body part 1 (Fig. 4) whereafter the cylindrical smooth part of the front body part 9 (Fig. 4) is housed in the halves of the rear body part 14 (Fig. 4) and the cylindrical protrusion 18 (Fig. 5, Fig. 6) with point elements 19 (Fig. 5, Fig. 6) is housed in the cylindrical slot 11 (Fig. 5) with point elements 12 (Fig. 5) formed in the cylindrical smooth part 9 of the front body part (Fig. 5), wherein the halves of the rear body part are connected similarly to those of the front body part using tapered protrusions 22 (Fig. 5, Fig. 6) and matching tapered holes 23 (Fig. 5, Fig. 6) in the rear body part 14 (Fig. 4) to form thereby an overlapping connection between the front body part 1 (Fig. 4) and the rear part 14 (Fig. 4) of the body.

## Claims

1. A body of a device for positioning a coronary stent or a coronary balloon within coronary arteries, said body comprising a retainer with lugs, a runner having a hole for receiving a delivery system, with protrusions for engaging with an internal thread in a cylindrical rear body part and with protrusions for moving along guides inside the body, on the one side, and with a cap having a hole for receiving the delivery system, with a rubber bush having a hole for receiving the delivery system with a cone-shaped and end surface inside matching the same surfaces inside the cap and the runner, housed in the cap with a thread for threading on the runner, on the other side, said body consisting of a front body part and a rear body part each formed of two halves, characterized represents, on the one side, a truncated part having a tubular extension for connection to a Y-connector and with a cutout for a coronary guide, and, on the other side, successively passes into a cylindrical part with holes for the retainer lugs with two point protrusions in each, into a cylindrical smooth part of a smaller diameter having a cylindrical slot with point elements formed at its end on the side of a runner guide, and into a runner guide, and the cylindrical rear body part is formed as a smooth surface on the inside with point elements formed circumferentially on the retainer side and as a circumferentially double-threaded surface, and a cylindrical protrusion with point elements is formed between the smooth inside surface and the circumferentially double-threaded surface of the rear body part, the outside rear body part is provided with horizontally extending smooth transparent stripes alternating with pebbled surface stripes, a chamfer is housed on the end surface of the rear body part, wherein a through-hole for introducing the coronary stent or coronary balloon delivery system inside the device is formed in the front body part and in the end surface of the rear body part.

2. The body device according to claim 1, **characterized in that** the truncated front body part is formed without tubular extension and is not intended for connecting to the Y-connector.

3. The body device according to claim 1, **characterized in that** the internal thread in the rear body part is formed as an offset single-thread.

4. The body device according to claim 1, **characterized in that** the outside surface of the rear body part is formed as a continuously pebbled surface.

5. A body of a device for positioning a coronary stent or a coronary balloon within coronary arteries, said body a retainer with lugs, a runner having a hole for receiving a delivery system, with protrusions for engaging with an internal thread in a cylindrical rear body part and with protrusions for moving along guides inside the body, on the one side, and with a cap having a hole for receiving the delivery system, with a rubber bush having a hole for receiving the delivery system inside with cone-shaped and end surfaces matching the same surfaces inside the cap and the runner, housed in the cap with a thread for threading on the runner, on the other side, said body consisting of a front body part and a rear body part each formed of two halves, wherein when assembling the device and first accommodating the delivery system, the rubber bush with the hole for receiving the delivery system and the cap with the hole for receiving the delivery system are first successively housed in the retainer on the thickened side and the runner with the hole for receiving the delivery system is housed on the opposite side of the retainer and the cap with the rubber bush inside is threaded into said hole whereafter a tube is introduced into the through-hole formed in the bush, cap and runner for arranging therein the coronary stent or coronary balloon delivery system, **characterized in that** the retainer comprising the rubber bush, the cap and the runner is housed inside the front body part with the thickened part of the retainer accommodated in the cylindrical body part with side holes for the retainer lugs and the rest of the retainer accommodate in the cylindrical smooth part of the front body part accommodated together with the runner in the rear body part, a cylindrical protrusion with point elements on inside rear body part is housed in a cylindrical slot with point elements in the cylindrical smooth part of the front body part on retainer side to form an overlapping connection between the front and the rear body parts when once the halves are connected.
